# EUROPEAN PATENT SPECIFICATION

(11) **EP 0 304 971 B1**
(45) Date of publication and mention of the grant of the patent: **04.02.1998**
(21) Application number: 88201179.4
(22) Date of filing: 12.08.1983
(51) Int. Cl.: C12N 15/00, C12P 21/02, A61K 38/55

(54) **A method of producing a polypeptide having the protease inhibition activity of mammalian alpha-1-antitrypsin**
Verfahren zur Herstellung eines Polypeptids mit der Proteasehemmenden Aktivität von Säugetier-alpha-1-Antitrypsin
Procédé de production d'un polypeptide ayant la même activité d'inhibition des protéases que l'alpha-1-antitrypsine des mammifères

(30) Priority: 13.08.1982 US 408099; 28.04.1983 US 489406
(43) Date of publication of application: 01.03.1989
(62) Divisional of application: 83304668.3
(73) Proprietor: ZymoGenetics, Inc., Seattle Washington 98102 (US)
(72) Inventor: Kawasaki, Glenn Hitoshi, Seattle Washington 98112 (US); Woodbury, Richard Grant, Seattle Washington 98155 (US)
(74) Representative: Allard, Susan Joyce

(56) References cited:
- EP-A- 0 114 777
- EP-A- 0 137 633
- BE-A- 895 961
- US-A- 4 559 311
- FEBS LETTERS, vol. 130, no. 2, August 1981, pages 297-300, Elsevier/North Holland Biomedical Press, NL; J. CARLSON et al.
- NATURE, vol. 297, 24th June 1982, pages 655-659, Macmillan Journals Ltd; M. LEICHT et al.

## Description

The ability to obtain expression of foreign, i.e., exogenous, DNA in unicellular microorganisms provided the opportunity to conveniently prepare long polypeptide chains of interest. Almost immediately, varied polypeptides, such as the small hormone somatostatin and more sophisticated polypeptides, such as insulin, interferons, thymosin and a variety of Vaccines having capsid proteins, were prepared and reported in the literature. For the most part, the initial work was performed in the bacterium E. coli which had been the subject of intensive study because scientists were familiar with many aspects of its genetic structure and properties. Initial attention was therefore directed to producing foreign proteins in E. coli. Once the ability to employ E. coli as a host was established, the limitations and disadvantages of employing E. coli encouraged the use of other hosts.

One host which appeared to be particularly attractive because it lacked many of the shortcomings of E. coli was yeast. However, yeast is a eukaryote and, therefore, has a more sophisticated genetic system. Furthermore, less is known about the yeast genome than is known about E. coli. In order to use yeast as a host for the production of proteins foreign to yeast, a number of discoveries are required, and new materials must be made available.

Initially, a replication system was required which provided stability in yeast, either as an extrachromosomal element or by integration into the yeast chromosome. In addition, the regulatory functions concerned with transcription and expression had to be developed in order to allow for expression of the desired protein. There was also the uncertainty whether foreign DNA sequences would be transcribed and translated and, if expressed, whether the resulting polypeptides would survive in the yeast cell. Also remaining to be determined was the effect of the foreign proteins on the viability of the yeast cell, such as the effect of recombinant DNA (RDNA) on mitosis, sporulation and vegetative growth.

There have, therefore, been substantial efforts to develop novel RDNA systems in yeast, which will allow for regulated expression of a protein of interest, as well as highly efficient production of such proteins.

Hitzeman et al., J. Biol. Chem., 255:12073-12080 (1980) describe a plasmid having a yeast 3-phosphoglycerate kinase (PGK) gene and accompanying regulatory signals capable of expression in yeast. Other references of interest include Clifton, et al., Genetics, 88:1-11 (1978); Clark and Carbon, Cell, 9:91-99 (1976); Thomson, Gene, 1:347-356 (1977); Holland and Holland, J.Biol. Chem., 254:5466-5474 (1979); Holland and Holland, ibid. 254:9830-9845 (1979); Nasmyth and Reed, Proc. Nat. Acad. Sci., 77:2119-2123 (1980); Broach, et al., Gene, 8:121-133 (1979); and Williamson, et al., Nature, 283:-214-216 (1980).

In the accompanying FIGURES:

FIGS. 1A and 1B are cDNA sequences of two forms of genes coding human alpha-1-antitrypsin.

FIG. 2 illustrates the restriction maps of plasmids CTEA32 and CAT1.

FIG. 3 is a diagram of the electrophoresis chromatogram showing purified alpha-1-antitrypsin produced according to the present invention.

FIG. 4 illustrates the restriction map of a plasmid C1/1.

FIG. 5 illustrates the DNA sequence of the multiple restriction site of pUC13.

FIG. 6 illustrates the restriction map of plasmid pUCal containing the DNA sequence from FIG. 1.

FIG. 7 is the restriction map of plasmid HAT4.

The present invention provides a method of producing a polypeptide having the protease inhibition activity of human alpha-1-antitrypsin, comprising the steps of transforming a yeast culture with a DNA transfer vector, said vector comprising a segment coding for human alpha-1-antitrypsin and a promoter capable of controlling expression of said polypeptide in said yeast culture, and growing said yeast culture under conditions suitable for expression of said polypeptide.

The present invention also provides a method of hyperproducing a polypeptide having the protease inhibition activity of human alpha-1-antitrypsin, comprising the steps of transforming a yeast culture with a DNA transfer vector, said culture comprising yeast cells which contain allelic mutations to GK-100 (ATCC 20669) mutations said vector comprising a segment coding for human alpha-1-antitrypsin and a promoter capable of controlling expression of said polypeptide in said yeast culture and growing said culture under conditions suitable for expression of said polypeptide.

Preferably the cells of the yeast culture comprise cells of the mutant strain GK-100 (ATCC 20669). The DNA transfer vector may comprise 2-micron plasmid DNA.

The present invention also provides for the steps of extracting the alpha-1-antitrypsin polypeptide produced and purification thereof.

The method of the present invention may also provide the step of altering a suitable vector to include said segment coding for human alpha-1-antitrypsin to form said DNA transfer vector.

The polypeptide produced by the present invention may comprise the amino acid sequence of naturally-occuring human alpha-1-antitrypsin. The polypeptide may be the predominant human form of alpha-1-antitrypsin.

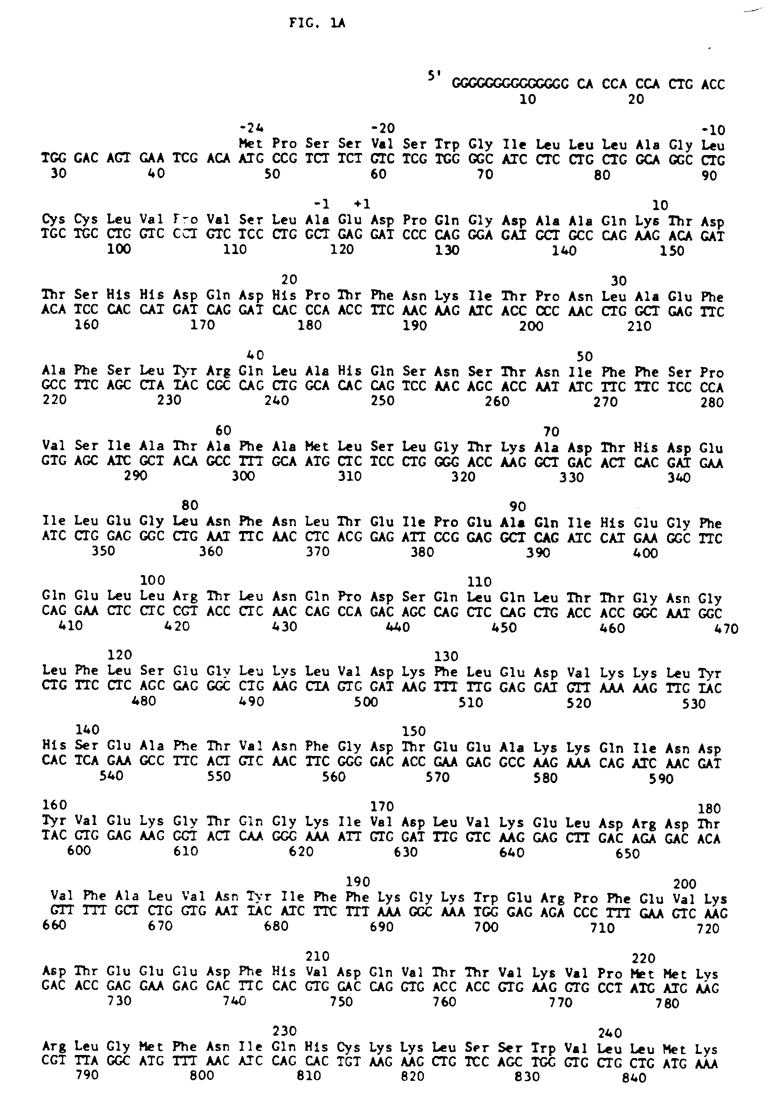

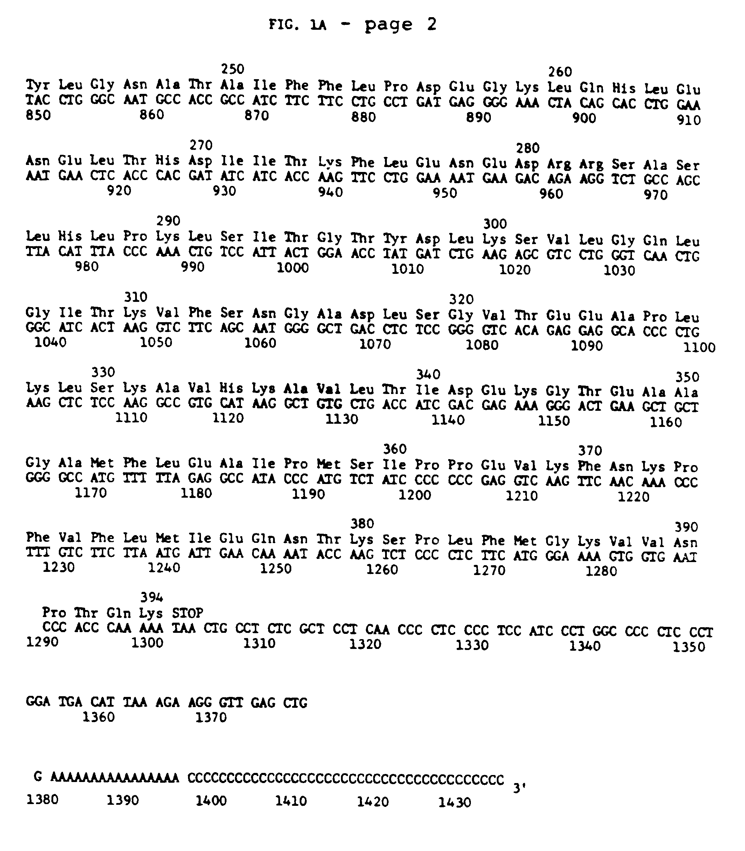

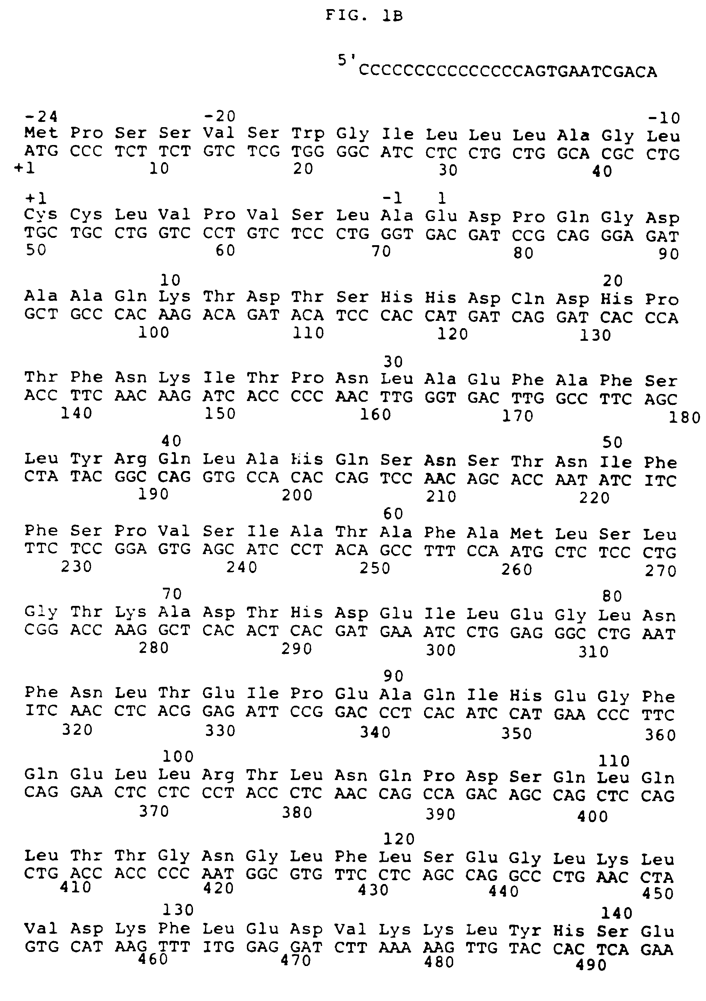

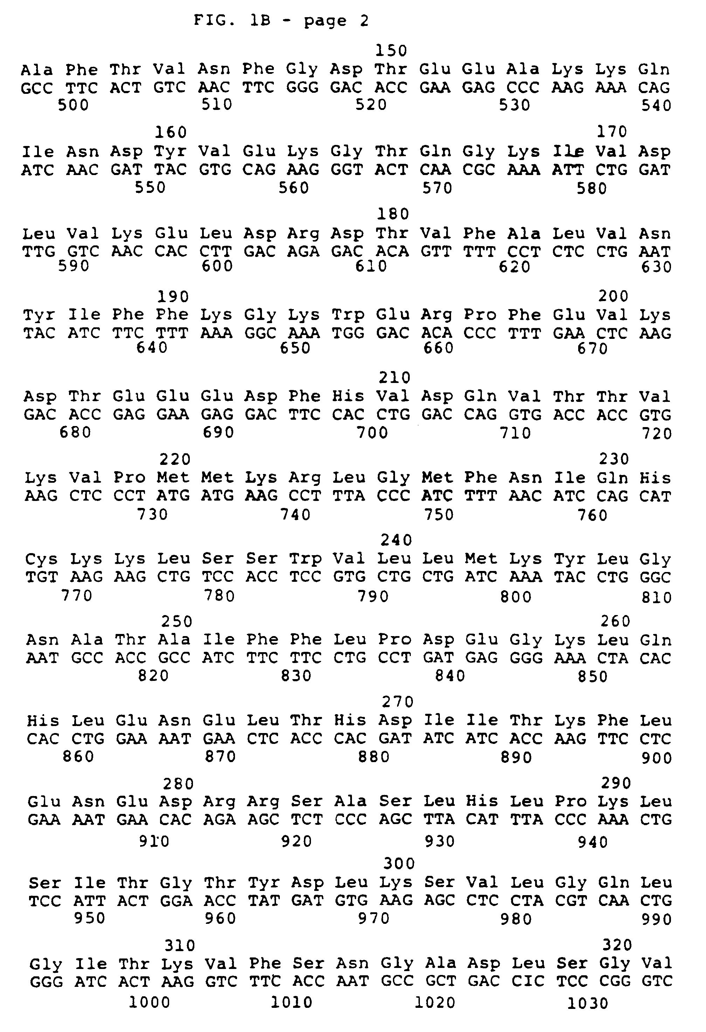

The present invention also provides for an unglycosylated human alpha-1-antitrypsin consisting essentially of an N-terminal methionine residue followed by the amino acid sequence shown in Fig 1A or 1B from amino acid 1 (Glu) to amino acid 394 (Lys) or unglycosylated mature human alpha-1-antitrypsin, said alpha-1-antitrypsin having the biological activity of the glycosylated form of human alpha-1-antitrypsin. These forms of unglycosylated human alpha-1-antitrypsin may be introduced into a host having a deficiency of alpha-1-antitrypsin or for modulating proteolytic activity in a mammalian host. In particular the alpha-1-antitrypsin can be administered to replace alpha-1-antitrypsin inactivated by tobacco and other smoke.

Promoters for use in expressing a polypeptide having the protease inhibition activity of human alpha-1-antitrypsin may be obtained by employing a gene bank having large fragments of DNA. By introducing the fragments into appropriate vectors, particularly shuttle vectors having replicons for prokaryotes and yeast, one can readily amplify and clone the yeast DNA in a bacterium and then introduce the yeast DNA into mutant yeast cells for complementation. In this manner, yeast fragments can be identified which complement auxotrophic lesions or mutations in a yeast host.

Having established a DNA segment having the desired gene for transformation into yeast host cell, one may-reclone by various techniques to shorten the DNA segment and provide for a segment which is primarily the gene of interest in conjunction with its regulatory signals for transcription and expression.

In order to retain the promoter, it is essential that the initiator methionine be determined and this codon be used for developing the strategy for introducing the alien DNA downstream from the promoter. Various techniques can be employed for providing a site for introduction of the alien DNA so as to be under the regulatory control of the promoter.

Where a restriction site is conveniently adjacent to the initiator methionine codon, the gene may be cleaved at that site and the DNA chewed back with Ba131 for varying periods of time, so as to chew into or past the initiator methionine codon or retain the initiator methionine codon.

Where there is no convenient restriction site, other splicing techniques such as primer repair may be employed. Also, by employing in vitro mutagenesis, one can introduce a restriction site adjacent the initiator methionine, which encodes for the initial amino acids of the desired protein. In each instance, a linearized DNA segment is obtained having the intact promoter and normally including other DNA sequences, such as an intact replicon, one or more markers, and the like.

Exemplary of the above procedure is the development of a vector having the promoter for the TPI1 gene. An exemplary vector CV13 having the replicons or replication systems from pBR322 and 2µ-plasmid of yeast, as well as the LEU2 gene was employed for insertion of a yeast fragment which was shown to have the TPI1 gene. This was achieved by employing double selection with a mutant yeast which was leu⁻, tpi⁻. The TPI1 gene was found to have a unique KpnI site. The vector was cleaved at the KpnI site and then treated with the double stranded exonuclease Bal31 for varying times to chew back the DNA to about the f-met codon. Linkers were then inserted providing desired restriction sites. Alien DNA could then be inserted providing a sequence having a f-met codon in the appropriate position for initiation. Alternatively, the foreign DNA can be expressed using the f-met codon of the TPI1 gene.

In order to obtain expression, an extrachromosomal element construct will be prepared having a number of sequences defining different functions. One function is the replication system, which forms part of a vector. Another function is a promoter by itself or in conjunction with the alien DNA. Other functions include initiators and terminators of expression. Also, there will be selectable markers.

In developing an appropriate vector, while not necessary, it will be common to have both a replication system for yeast and a replication system for a prokaryote (a shuttle vector). The replication system for yeast may be one which provides for stable maintenance of an extrachromosomal element or one which provides a sufficient lifetime for the DNA in the host, that there is an acceptable probability of integration of the DNA into the host. Integration can be greatly aided by providing for a sequence homologous to the host DNA, so as to provide for recombination. Generally, the homologous sequence will be at least about 800bp usually not more than about 2000bp. Therefore, either integration or an autonomous replication system, such as the use of the ARS1 gene, may be employed to provide for the maintenance of the alien DNA in the yeast host. The replication system which is chosen should provide for a reasonable copy number usually greater than 1, preferably greater than 5. A wide variety of replication systems are available on a wide variety of prokaryotic vectors, such as pBR322, pACYC184, pSC101, pMB9, etc. Alternatively, one or more copies of the DNA construct can be integrated into the host chromosome. The replication systems may also be conditionally regulated, usually being temperature sensitive so that replication can be turned on and off by varying the temperature.

In addition to the replication system, there will also be one or more selectable markers, there usually being at least one marker in addition to the alien DNA, which may serve as a marker. Conventional markers include biocidal markers providing antibiotic resistance and those providing resistance to toxins and heavy metal. Also useful is employing an auxotrophic host and providing prototrophy by complementation. In addition to the conventional selection systems just described, the glycolytic genes of the present invention are particularly desirable markers since they can provide for selection, using sugars as selective substrates, in appropriate mutant host strains.

Other genes may also be inserted into the extra-chromosomal element for a variety of purposes. Where integration is desirable in the genome of the host, a homologous sequence for a particular region of the host genome may be included in the extrachromosomal element. Where amplification of one or more sequences is desired, genes known to provide such amplification, such as dihydrofolate reductase genes, which respond to methotrexate stress or metallothionein genes, which respond to heavy metal stress, may be included in the extrachromosomal element, flanked by the DNA regions to be reiterated. Other regulatory signals may also be included, such as centromeres, autonomously replicating segments, etc.

In order to isolate the promoters of interest, clones can be made of yeast chromosomal DNA by random digestion or mechanical shearing of the yeast genome. The presence of the desired gene is then determined by introducing a homogeneous clone of a yeast fragment into an auxotrophic host for complementation. Desirably, the cloning vehicle may have another gene which allows for an additional basis for selection, so that double selection techniques can be used. The mutants are substantially incapable or growing on limited nutrient medium, so that one can select for the presence of the desired glycolytic gene by the choice of medium. After isolating the yeast fragment having the desired gene, the fragment may be subcloned so as to remove superfluous DNA flanking regions and provide for a fragment which is more easily manipulated. The smaller fragment containing the desired gene, of a size less than about 500 base pairs may then be further cloned, restriction mapped and sequenced, so as to provide a useful source for the desired promoters and insertion of the alien DNA. Also, as indicated, the promoters in themselves may be useful, in acting as a titrater for repressor or activator, where it is desirable to modulate the production of a particular enzyme in the yeast host. The alien DNA may be from any source, either naturally occurring or synthetic, either prokaryotic or eukaryotic. Of particular interest are mammalian genes which express a poly(amino acid), that is, polypeptide or protein which has physiological activity. To varying degrees, poly(amino acids) prepared in yeast may be modified by glycosylation, where the glycosylation may not occur or may occur at different sites from the naturally occurring mammalian polypeptide and/or in different degrees with different saccharides. It is therefore of great interest to be able to prepare polypeptides which are different from the naturally occurring polypeptide by the degree and manner of glycosylation and in many instances may differ in one or more ways as to the amino acid sequences, where there may be deletions of one or more amino acids or substitutions of one or more amino acids. Mammalian genes may come from a wide variety of mammalian sources, such as domestic animals (e.g. bovine, porcine, ovine and equine) and primates e.g. humans and monkeys.

A DNA construct containing the gene for human alpha-1-antitrypsin and a yeast triose phosphate isomerase promoter fragment is described and made.

The protease inhibitor has the same or substantially the same amino acid sequence of human alpha-1-antitrypsin and is capable of inhibiting a number of proteolytic enzymes. The human alpha-1-antitrypsin gene appears to reside within a 9.6 kb EcoRI DNA fragment in the human genome. The mature mRNA appears to have about 1400 nucleotides. One human alpha-1-antitrypsin cDNA has the sequence shown in FIG. 1B. The predominant form of human alpha-1-antitrypsin is shown in FIG 1A. Other naturally-occurring forms (polymorphisms) are known.

The sequencing of chromosomal DNA coding for alpha-1-antitrypsin (or hereinafter in the alternative 'AT') has been described by Kurachi et al., Proc. Natl. Acad. Sci. U.S.A., 78, 6826-6830 (1981) and by Chandra et al., Biochem. Biophys. Res. Comm., 103, 751-758 (1981), the disclosures of which are incorporated herein by reference. A primate gene for alpha-1-antitrypsin may be obtained by DNA cloning methods described by Chandra et al., ibid. The gene coding for the predominant form of human alpha-1-antitrypsin, isolated from a human cDNA library by using the baboon sequence as a DNA hybridization probe is shown in FIG. 1A.

The human alpha-1-antitrypsin has a BamHI restriction site which allows the cutting of the gene with the removal of information for a single glutamic acid from the mature protein. Various schemes can be employed for introducing the human alpha-1-antitrypsin gene adjacent the glycolytic promoter to be under the regulation of the promoter. Where the promoter does not have a convenient restriction site near the f-met codon, the glycolytic gene may be cleaved and chewed back to the promoter with Bal31. A linker may then be introduced downstream from the promoter to provide a convenient cohesive end or flush end for joining to the human alpha-1-antitrypsin gene. The linker can also provide one or more codons for amino acids at the N-terminus of the alpha-1-antitrypsin gene, which may be the same or different from the naturally occurring amino acids.

The gene for human alpha-1-antitrypsin may then be inserted into the extrachromosomal element downstream from the glycolytic promoter, where an f-met codon is provided for initiation of expression of the human alpha-1-antitrypsin.

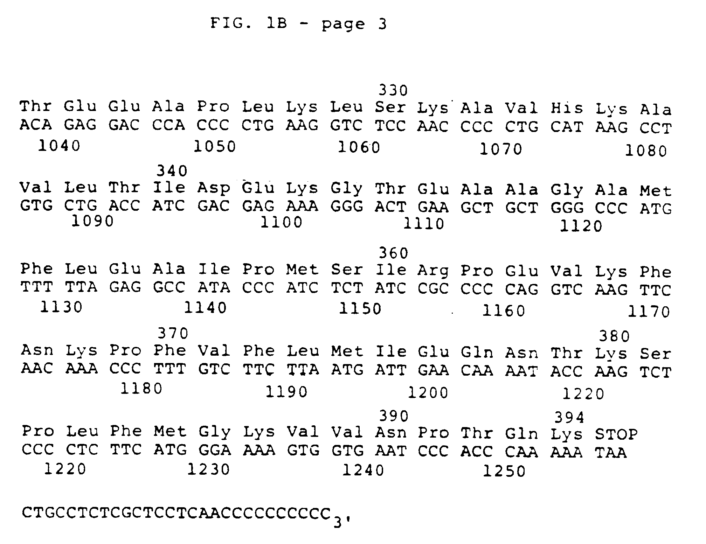

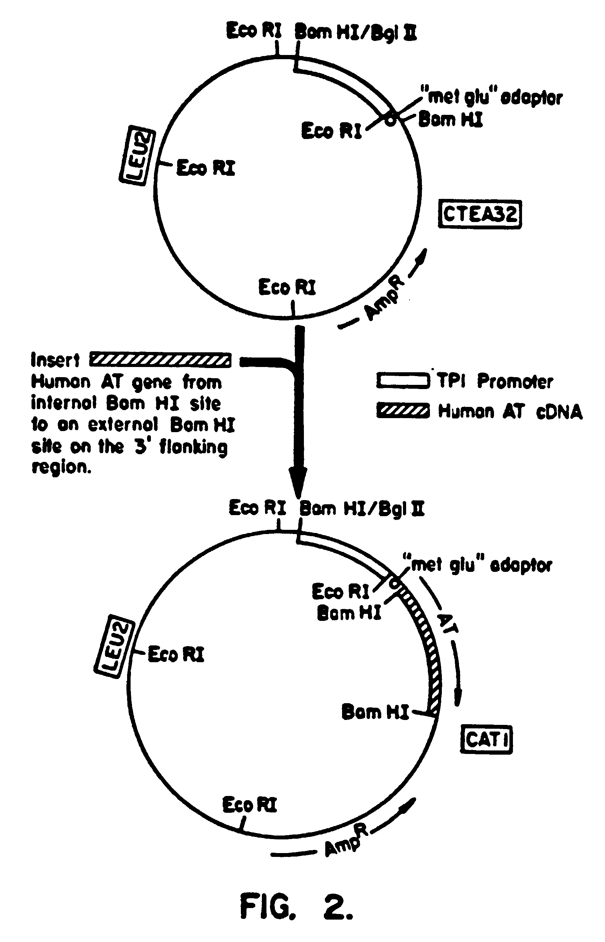

For example, the cDNA coding for alpha-1-antitrypsin may then be inserted into an expression vector, such as CTEA32 (FIG. 2), which contains the yeast promoter for triose phosphate isomerase (TPI) inserted at the BamHI site of the shuttle plasmid, CV13 [Broach J.R., Strathern J.N., Hicks J.B., Gene, 8:121-133 (1979)]. A synthetic DNA adaptor was ligated into the TPI promoter after the TPI structural sequences were removed by BAL31 digestion from the KpnI restriction site within the TPI coding region. (Alber et al, J. Molec. Applied Genet., 1, 419-434 (1982)). This adaptor contained an ATG codon for translation initiation, followed by the sequence GAGGATCC. The GAG codon specifies a glutamic acid residue, which is the first amino acid of the naturally-occurring human AT. The GGATCC portion of the adaptor is a cutting site for BamHI endonuclease and allows for the splicing of the remainder of human AT DNA sequence into this vector.

The BamHI site of CTEA32 was constructed to be "in frame" with the rest of the AT structural gene, thereby allowing for the expression of the polypeptide when a BamHI fragment from the cloned cDNA is appropriately inserted into CTEA32. The plasmid consisting of CTEA32 plus the AT gene is called CAT1 (FIG. 2).

This DNA construct containing the gene for human AT located downstream to a yeast triose phosphate isomerase (TPI) promoter fragment was transformed into yeast strains, N501-B and GK100. Transformation into yeast is described by Beggs, Nature, 275, 104-109 (1978). Screening of the transformed yeast strains by immunological assays (competition assays and ELISA assays, using antibodies against alpha-1-antitrypsin) confirmed the presence of large amounts of human AT in yeast made from the plasmid CAT1. The "wild-type" yeast strain, N501-1B (described by Kawasaki et al., Biochem. Biophys. Res. Comm., 108, 1107-1112 (1982)), when transformed with CAT1, produced 1.8 mg alpha-1-antitrypsin per gram of soluble protein (or 0.18% alpha-1-antitrypsin), when grown at 30° on a synthetic minimal medium (modified Wickerham's medium) with 6% glucose. A mutant yeast strain, GK100, when transformed with CAT1, produced 10-15 mg alpha-1-antitrypsin per gram soluble protein (or 1-1.5% alpha-1-antitrypsin) under the same growth conditions. Strains N501-1B and GK100 each carry a defective LEU2 gene which allows for the selective maintenance on minimal and leucine-less media of CV13 and CV13-derived plasmids (such as CAT1) which each contain a functional LEU2 gene. When grown on minimal media with only CV13 as a control, N501-1B and GK100 produce no detectable AT. Thus, AT may be specifically produced by the CAT1 plasmid.

Since GK100 produces significantly more AT than N501-1B, it is preferred. However, the present invention is not limited to AT production by GK100. It may be desirable to utilize mutations in GK100 which lead to hyperproduction of AT.

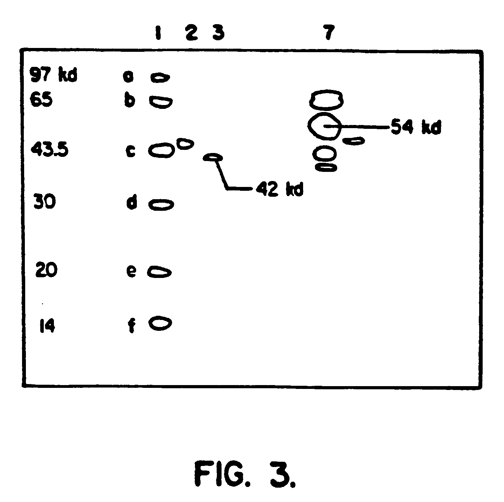

An immuno-adsorption column, made according to the method of Cuatrecasas, P. J. Biol. Chem., 245, 3059 (1970), was prepared by covalently attaching affinity-purified goat antibodies to human AT to CNBr-activated Sepharose. Disrupted GK100 yeast cells were extracted with 3 volumes of phosphate buffered saline pH 7.2 containing 0.5M NaCl, and the extracts were applied to the column. Yeast produced human AT (0.5-1.0 mg) was eluted from the column with 3M NaSCN. After the material was dialyzed to remove salt it was analyzed by electrophoresis on a polyacrylamide gel in the presence of sodium dodecyl sulfate, the results of which are shown in FIG. 3. Based on the relative migration of the protein in the gel, the approximate molecular weight of the human alpha-1-antitrypsin made in yeast is 42,000-43,000 daltons. Naturally occurring human AT has a molecular weight of approximately 54,000 daltons, having a carbohydrate composition of approximately 16% by weight, as shown by Hodges et al, J. Biol. Chem., 254, 8208-8212 (1979). It therefore appears that the yeast produced AT may be unglycosylated or substantially unglycosylated and may lack carbohydrate portions present in the naturally occurring protein.

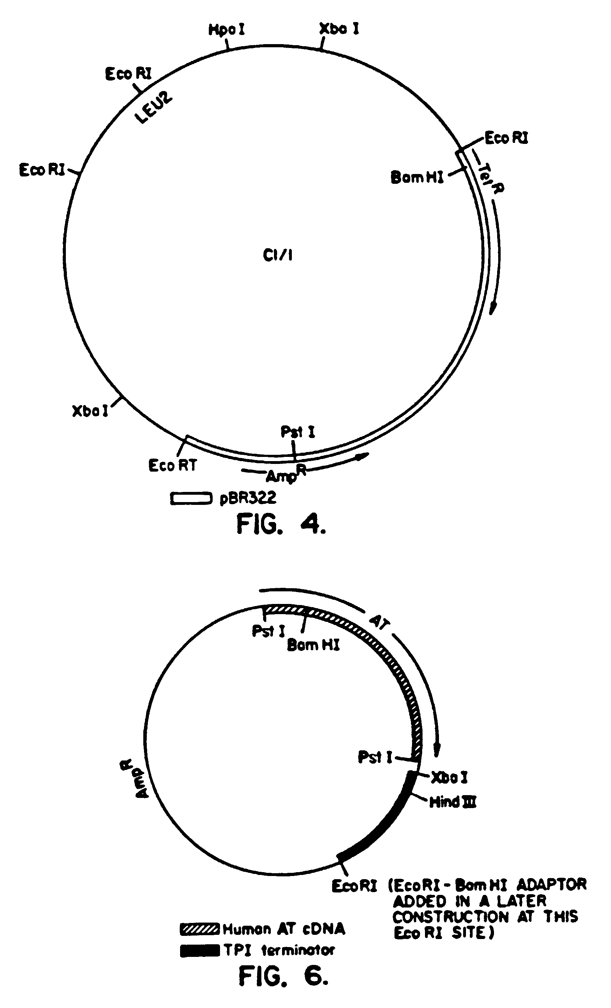

Alternatively, other expression vectors may be constructed which contain a segment coding for alpha-1-antitrypsin. Such expression vectors may be constructed by methods known to those of ordinary skill in the art using available DNA constructs. A preferred vector is plasmid C1/l, which is more stable than CV13 and CV13 derived vectors, such as CAT1. C1/l was constructed from plasmid, pJDB248 (Beggs, J., Nature, 275, 104-109 (1978)). The pMB9 sequences were removed from pJDB248 by partial digestion with Eco RI and were replaced by pBR322 DNA which was cut with Eco RI. The restriction map of C1/l is given in FIG. 4. The C1/l plasmid contains the entire 2-micron DNA from yeast (S.cervisiae), with a pBR322 insertion at an EcoRI site. It also contains the LEU2 gene. Thus, the yeast TPI promotor with the adaptor may be inserted into the single BamHI site in the Tc^{R} gene of C1/l. Then the AT sequence, attached to a transcription terminator fragment from the yeast TPI gene, may be inserted into the BamHI side downstream from the TPI promotor. The resulting plasmid, HAT4, may then be transformed into N501-1B and GK100 in a manner as described above.

The sequence of the first ten amino acids in the yeast-produced AT may be confirmed by amino acid sequence analysis as identical to the first ten amino acids of the naturally occurring human AT:

      1   2   3   4   5   6   7   8   9   10
H2N-Glu-Asp-Pro-Gln-Gly-Asp-Ala-Ala-Gln-Lys-

The yeast-produced AT does not contain the initiation methionine which is specified by the ATG start codon. Therefore, the yeast cell processes off the methionine to produce the amino acid sequence of natural human AT.

The polypeptides produced according to the present invention having AT activity may be useful for treatment of a genetic AT deficiency and other diseased states related to inadequate levels of AT. Thus, conditions such as emphysema and other lung disorders related to progressive digestion of lung sacs may be treated, such as, chronic obstructive pulmonary disease or adult respiratory distress syndrome. Non-genetically related emphysema may also be treated, such as, emphysema resulting from heavy smoking. Conditions not necessarily confined to the lungs may also be treated, such as, cystic fibrosis and arthritis. For a review of AT deficiency, see Gadek, J.E., and R. Crystal, "Alpha-1-Antitrypsin Deficiency", The Metabolic Basis of Inherited Disease, J.B. Stanbury, J.B. Wyngaarden, D.S. Fredrickson, McGraw-Hill, N.Y. pp. 1450-67 (1982).

The alpha-1-antitrypsin can be used as an antigen for production of polyclonal and monoclonal antibodies to human alpha-1-antitrypsin, for introduction into a host having a deficiency of alpha-1-antitrypsin, or for modulating proteolytic activity in a mammalian host. In particular, the alpha-1-antitrypsin can be administered to humans to replace alpha-1-antitrypsin which has been inactivated (oxidized) by tobacco and other smoke.

The polypeptides according to the present invention may be admixed with conventional pharmaceutical carriers. Preferably, the polypeptides are to be administered intravenously or by inhalation. While the effective dosages may vary according to the severity of the condition and weight of the subject, dosages in the range of 0.5-10.0 gm/week of a polypeptide introduced intravenously may, in many cases, be effective. Lower dosages may be effective if the method of administration is by inhalation. Oral administration may also be effective provided the AT is protected in capsules or coated carriers from premature degradation in the digestive tract.

The following examples 1, 2 and 3 are not specific examples of the present invention. They describe experiments to demonstrate transformation of yeast cells with vectors which may contain the sequence coding for human alpha-1-antitrypsin. Examples 4, 5 and 6 are examples of the present application.

### EXAMPLE 1 - not an example of the invention

Strains. Isogenic strains carrying mutations in PGI1, PGK1, GPM1, PYK1, and GCR1 where obtained by ethyl methane sulfonate (EMS) mutagenesis of S. cerevisiae (S. c.) X2180-1A (MATa SUC2 CUP1 gal2, from the Berkeley Yeast Stock Center). 35,000 independent colonies were grown on YEP-3% glycerol-2% ethanol and were screened by replica plating for the inability to grow on YEP-4% dextrose (Table 1).

Identification of specific lesions was made by complementation tests with known glycolysis mutants (Ciriacy and Breitenbach, J.Bacteriol, 139:152-60 (1979)), while at least 15 additional complementation groups were found by intercrossing mutant strains. Enzyme assays (Clifton et al. Genetics, 88:1-11 (1980)) confirmed the glycolytic defects in pgi1, pgk1, gpm1, pyk1, and gcr1 mutants.

A LEU2 mutant was also derived from S.cerevisiae X2180-1A by EMS treatment and was crossed to X2180-1B (an isogenic MATa strain) to produce N501-1B (MATα leu2 SUC2 CUP1 gal2). Cycloheximide (cyh2) and canavanine (can1) resistances were then selected as spontaneous mutations in N501-1B. The glycolysis mutants were crossed to N501-1B to produce a series of isogenic leu2 strains each defective in a single glycolytic function or in GCR1.

A tpil mutant, S. cerevisiae GLU77 was crossed to N551-1A (MATa leu2 SUC2 CUP1 gal2); strains derived from this mating were crossed twice to N501-1B to produce a tpi1 leu2 strain, N587-2D, which was similar in genetic background to the other Glycolysis mutants.

Mutations in three glucose phosphorylating enzymes produce a strain which is unable to grow on dextrose as the sole carbon source and which is resistant to catabolite repression by 2-deoxyglucose and glucosamine. N517-6C (hxk1 hxk2 glk1 leu2 can1-100 cyh2 ade2-1) was derived from a hxk1 hxk2 glk1 strains, D308.3, by screening for glucosamine- resistant spore colonies. Defects in glucose kinasing activities were confirmed by assay.

**TABLE 1**

| Complementation Groups of glu⁻ Derivatives of X2180-1A | | |
|---|---|---|
| Gene | No. of Mutants | |
| PYK1 | 14 | |
| PDC1 | 9 | |
| GCR1 | 4 | |
| PGI1 | 3 | |
| GPM1 | 3 | |
| PGK1 | 1 | |
| TPI1 | 0 | |
| FDP | 0 | |
| (LEU2) | (1) | |
| | | |
| I | 11 | |
| II | 10 | |
| III | 3 | |
| IV | 5 | |
| V | 1 | |
| VI | 1 | |
| VII | 2 | |
| VIII | 3 | 60 other mutations not in the complementation groups |
| IX | 2 | |
| X | 3 | |
| XI | 2 | |
| XII | 1 | |
| XIII | 1 | |
| XIV | 5 | |
| XV | 1 | |
| 27 sterile glu⁻ strains 35,000 colonies screened (EMS mutagenized for 50% kill) | | |

The homothallic diploid strain, S. c. AB320 was the source of the yeast DNA pool (Nasmyth and Reed, Proc. Nat. Acad. Sci., 77:2119-2123 (1980) and was used as a control in some experiments.

The triose phosphate isomerase gene (including the upstream sequence having the regulatory signals) is as follows:

The pyruvate kinase gene upstream sequence having the regulatory signals is as follows:

10 20 30 40 50 60
GAATTCACCA TCATACCTAC GTAAATGTGT TCCGCACCGT CACAAACTGT TTTCTACTGT
CTTAAGTGGT AGTATGGATG CATTTACACA AGGCGTGGCA GTGTTTGACA AAAGATGACA

70 80 90 100 110 120
TCTTTCTTCT TTCGTTCATT CAGTTCAGTT GACTCAGTGC TTTGTTCAAT GGATCTTAGC
AGAAAGAAGA AAGCAAGTAA GTCAAGTCAA CTGAGTCACG AAACAAGTTA CCTAGAATCG

130 140 150 160 170 180
TAAAATGCAT ATTTTTTCTC TTGCTAAATG AATGCTTGTG ATGTCTTCCA AGTGATTTCC
ATTTTACGTA TAAAAAAGAG AACGATTTAC TTACGAACAC TACAGAAGGT TCACTAAAGG

190 200 210 220 230 240
TTTCCTTCCC ATATGATGCT AGCTACCTTT ACTGTCTTCC TAAAAAAAAA AAAACGCTCG
AAAGGAAGGG TATACTACGA TCGATGGAAA TGACAGAAGG ATTTTTTTTT TTTTGCGAGC

250 260 270 280 290 300
CCATCAAAAC GATATTCGTT GGCTTTTTTT TCTGAATTAT AAATACTCTT TGGTAACTTT
GGTAGTTTTG CTATAAGCAA CCGAAAAAAA AGACTTAATA TTTATGAGAA ACCATTGAAA

310 320 330 340 350 360
TCATTTCCAA GAACCTCTTT TTTCCAGTTA TATCATGGTC CCCTTTCAAA GTTATTCTCT
AGTAAAGGTT CTTGGAGAAA AAAGGTCAAT ATAGTACCAG GGGAAAGTTT CAATAAGAGA

370 380 390 400 410 420
ACTCTTTTTC ATATTCATTC TTTTTCATCC TTTGGTTTTT TATTCTTAAC TTGTTTATTA
TGAGAAAAAG TATAAGTAAG AAAAAGTAGG AAACCAAAAA ATAAGAATTG AACAAATAAT

430 440 450 460 470 480
TTCTCTCTTG TTTCTATTTA CAAGACACCA ATCAAAACAA ATAAAACATC ATCACAATGT
AAGAGAGAAC AAAGATAAAT GTTCTGTGGT TAGTTTTGTT TATTTTGTAG TAGTGTTACA

490 500 510 520 530 540
CTAGATTAGA AAGATTGACC TCATTAAACG TTGTTGCTGG TTCTGACTTC AGAAGAACCT
GATCTAATCT TTCTAACTGG AGTAATTTGC AACAACGACC AAGACTGAAG TCTTCTTGGA

550 560 570 580 590 600
CCATCATTGG TACCATCGGT TCAAAGACCA ACAACCCAGA AACCTTGGTT GCTTTGACAA
GGTAGTAACC ATGGTAGCCA AGTTTCTGGT TGTTGGGTCT TTGGAACCAA CGAAACTGTT

610 620 630 640 650 660
AGGCTGGTTT GAACATTGTT CGTATGAACT TCTCTCACGG TTCTTACGAA TACCACAAGT
TCCGACCAAA CTTGTAACAA GCATACTTGA AGAGAGTGCC AAGAATGCTT ATGGTGTTCA

670 680 690 700
CTGTGGTTGA CAACGCCACA AACTCCGAAG AATTGTACCC
GACACCAACT GTTGCGGTGT TTGAGGCTTC TTAACATGGG

### Screening of clone bank.

The leu2 glycolysis mutants were transformed with a yeast DNA pool inserted into pYE13, a high copy plasmid carrying a selectable LEU2 wild-type gene (Broach et al., Gene, 8:121-133 (1979)). The glycolytic genes were obtained by complementation, involving the simultaneous selection for growth on glucose and leucine prototrophy. A synthetic medium containing yeast nitrogen base, 4% glucose, and the following supplements was used: per liter, 40mg adenine, 20mg arginine, 50mg aspartate, 10mg histidine, 60mg isoleucine, 40mg lysine, 10mg methionine, 60mg phenylalanine, 50mg threonine, 40 mg tryptophan, 50mg tyrosine, 20mg uracil, and 60mg valine.

The transformants were purified on leucineless media and were then grown on a non-selective medium (YEPGE) to allow mitotic segregation of the plasmids. Strains which cosegregated the leu2 and glycolysis mutant phenotypes, as determined by replica plating on selective media, were assayed for glycolytic enzyme activities. Yeast DNA preps were made, and the E. coli strain, RR1, was transformed, selecting for ampicillin resistance, to verify the presence of plasmid DNAs in these yeast glycolytic transformants.

### Enzyme Assays.

The transformed yeast strains were selectively grown on minimal medium with 8% glucose (adenine was added to a final concentration of 50 mg/1). The wild-type control, N501-1B, was grown on the same medium plus leucine (100mg/1). The glycolysis mutant strains were grown on YEP-5% glycerol-1% lactate. Overnight cultures were fed fresh media and were aerobically grown at 30° for four hours before harvesting. The cells were washed two times with water and resuspended in 50mM K₂HPO₄ 2mM EDTA 3mM 2-mercaptoethanol (adjusted to pH7.4 with HCl). Extracts were obtained by vortexing the cells with an equal volume of glass beads (.45 mm diam.) at high speed for two minutes. The cell debris was removed by centrifugation in a microfuge for 15 min. at 4°. Enzymes were assayed as described by Clifton and Breitenbach, supra. Protein concentrations were determined by the Biuret-TCA method.

### EXAMPLE 2 - Not an example of the invention

In order to determine the activity of the various glycolytic genes in the transformants, the various enzymes were assayed and the results for the transformants were compared to mutant and wild-type strains. The gcr1 mutant had 5-10% of the wild-type levels of most glycolytic activities (exemplified by PGI, aldolase and enolase) and grows very poorly on glucose media. In contrast, the GCR1 transformants had nearly wild-type levels of enzymes and were virtually identical to wild-type for growth on glucose media. The other glycolysis mutants had less than 5% of the normal levels of their respective enzyme activities. However, when transformed with a complementing high copy plasmid, the specific enzyme activities were substantially elevated above wild-type levels (typically 5-10 fold higher). The following Table 2 indicates the results.

**TABLE 2**

| Comparison of Glycolytic Activities in Wild-type, Mutant, and Transformed Strains | | | | |
|---|---|---|---|---|
| Enzyme | Activities | | | |
| | Wild-type^{a} | Mutant^{b} | Transformant^{c} | Ratio: Transf/Wt |
| PGI | 2.85 | .0065 | 31.49 (10) | 11.1 |
| TPI | 18.3 | .0000 | 167.8 (10) | 9.2 |
| PGK | 1.99 | .0046 | 17.67 (3) | 8.9 |
| GPM | 0.74 | .0000 | 4.80 (10) | 6.5 |
| PYK | 4.02 | .0057 | 14.77 (10) | 3.7 |

| | Wild-type^{a} | gcrl Mutant^{d} | GCR1 Transf^{c} | |
|---|---|---|---|---|
| PGI | 2.85 | .2436 | 2.42 (10) | .85 |
| Aldolase | 4.33 | .4415 | 2.96 (10) | .68 |
| Enolase | 0.43 | .0274 | .316 (10) | .74 |

| | | | | |
|---|---|---|---|---|
| ^{a}wild-type is N501-1B. | | | | |
| ^{b}The respective mutant strains are N543-9D (pgi1 leu2), N587-2D (tpi1 leu2), N548-8A (pkg1 leu2), N583-2C (gpm¹ leu2), and N549-3A (pyk1 leu2). | | | | |
| ^{c}The activities of the transformants are averages for many different isolates. The numbers in parentheses represent the numbers of independent transformants assayed. | | | | |
| ^{d}The gcr1 leu2 mutant strain is N525-2C. | | | | |

### EXAMPLE 3 - Not an example of the invention

In order to demonstrate that the hyperproduction of glycolytic enzymes was specific to the mutational defect complemented by the particular plasmid, assays for ten different glycolytic proteins were conducted on the various transformants. The following Table 3 reports the results for one transformant for each of the six different glycolysis genes which were examined in detail.

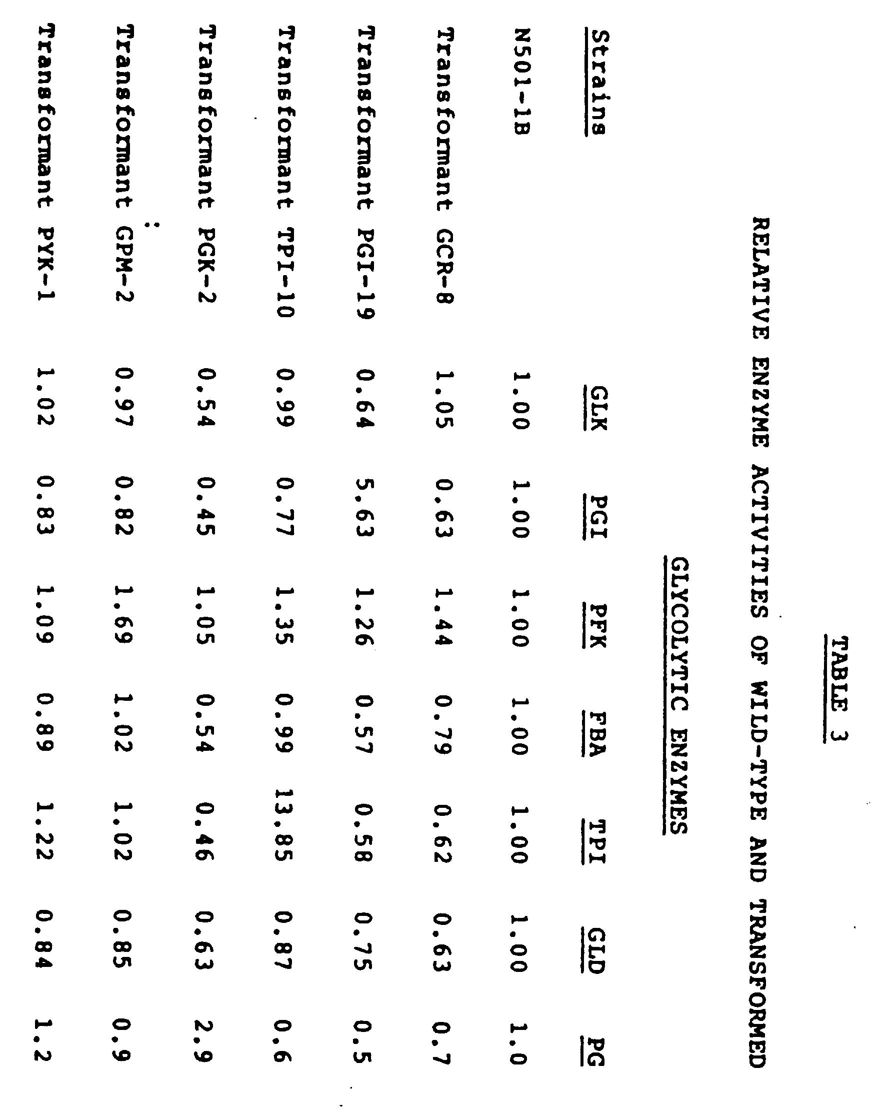

The GCR-8 transformant gave nearly wild-type levels of all ten enzymes, while PGI-19, TPI-10, PGK-2, GPM-2 and PYK-1 transformants overproduced their respective glycolytic proteins, but not other enzymes.

It was noted that the plasmids readily segregated (typically 5-50% segregation in fully grown cultures even under selective pressure of leucine prototrophy, so the assayed cultures probably contain cells with a range of number of plasmids. By complementation in E. coli and/or sequencing, TPI1 and PYK1 have both been shown to be the structural gene.

### EXAMPLE 4

Exploitation of the promoter for TPI1 for the production of human alpha-1-antitrypsin was demonstrated as follows. The plasmid CV13 was employed. CV13 can be maintained by selection of yeast with an average of about ten copies per cell. CV13 is comprised of pBR322, the replicon for the 2µ-plasmid and the yeast LEU2 gene. TPI1 promoter fragment was obtained by cutting the TPI1 gene at the unique KpnI site (bases 511 to 518); and the resulting linearized DNA was then treated with Bal31 for four to five minutes in order to remove the TPI1 structural sequences. Linkers, either EcoRI, Hind III or BamHI, were then inserted. The linkers will then be cleaved with the appropriate restriction enzyme to provide cohesive ends for insertion of human alpha-1-antitrypsin genes. The human alpha-1-antitrypsin gene was digested with BamHI, which cleaves at the 5'-terminus of the coding strand to remove the information for a single glutamic acid codon from the mature protein. Four different constructions were prepared, as set forth in the following Table 4. From this table it is noted that the glutamic acid codon is substituted by the codons for alanine and proline in three of the constructions having the initiator methionine.

After ligation of the human alpha-1-antitrypsin construction into the CV13 plasmid, the resulting plasmid was transformed into S. c. N501-1B. The resulting yeast cells were then grown on a minimal synthetic medium.

**TABLE 4**

| Plasmid | N-terminal amino acid | Orientation in CV13 |
|---|---|---|
| CAT1 | met glu + hAT* | clockwise |
| | | |
| C-Tα2 | met ala pro + hAT | counterclockwise |
| | | |
| C-Tα1 | met ala pro + hAT | clockwise |
| | | |
| C-TSα2 | met ala pro + hAT, but missing part of TPI promoter | counterclockwise |

| | | |
|---|---|---|
| *remainder of approximately 400 amino acids of human alpha-1-antitrypsin | | |

Yeast cells containing the human alpha-1-antitrypsin genes were broken open by vortexing with glass beads (0.45mm) at high speed for 2-3 minutes. The extraction buffer contained 50mM K₂HPO₄, 2mM EDTA, 2mM 2-mercaptoethanol and 1mM PMSF (pH7.4). Cell debris was removed by centrifugation and the extracts contain 3-4mg/ml protein as determined by Lowry assays.

The presence of human alpha-1-antitrypsin was determined using a RIA, employing tritium-labeled human alpha-1-antitrypsin and antibody directed against the protein. The following Table 5 indicates the results.

**TABLE 5**

| Competition assay for alpha-1 antitrypsin | | | | | |
|---|---|---|---|---|---|
| Plasmid | Tritium Counts | Average Count | α-1-AT [µg] | Total Protein (µg) | %Total Protein |
| CAT1 | 46010 | 49133.5 | 0.75 | 420 | .18 |
| | 52257 | | | | |
| | | | | | |
| C-Tα2 | 12268 | 12799 | 3.35 | 380 | .88 |
| | 13330 | | | | |
| | | | | | |
| C+Tα1 | 41635 | 40353 | 0.95 | 360 | .26 |
| | 39071 | | | | |
| | | | | | |
| C-TSα2 | 66490 | 68264 | 0 | 345 | 0 |
| | 70038 | | | | |
| | | | | | |

| Controls** | | Counts | | | |
|---|---|---|---|---|---|
| 0 µg α-1 | | 68440 | | | |
| 0.25 µg α-1 | | 65333 | | | |
| 0.5 µg α-1 | | 58928 | | | |
| 1.0 µg α-1 | | 38468 | | | |
| 2.0 µg α-1 | | 19559 | | | |
| 3.0 µg α-1 | | 14432 | | | |
| 4.0 µg α-1 | | 11155 | | | |
| 5.0 µg α-1 | | 9615 | | | |

| | | | | | |
|---|---|---|---|---|---|
| *Plasmids were grown in yeast strain, N501-1B. 100µl of extracts were assayed. **Non-radioactive alpha-1 antitypsin mixed with 100µl of yeast extract (330µg protein) | | | | | |

It is evident from the above results that an immunologically active product is obtained, which is capable of competing with naturally occurring human alpha-1-antitrypsin for antibodies to the native protein. Furthermore, the expression of the alpha-1-antitrypsin gene is regulated by the TPI promoter, for as is seen, where a portion of the TPI promoter is removed, no alpha-1-antitrypsin is produced. In addition, the production of the mammalian protein human alpha-1-antitrypsin has not been optimized in the above study, so that the results indicate a minimum production of product which can be further enhanced. Thus, the TPI promoter is found to be an effective promoter for efficiently producing high yields of expression products of alien DNA.

### EXAMPLE 5

### Purification Of Alpha-1-Antitrypsin From Yeast GK100 Yeast Extracts

An immuno adsorption column was prepared by covalently attaching affinity-purified antibodies to human alpha-1-antitrypsin to CNBr-activated Sepharose according to the method of Cuatrecasas, J. Biol. Chem., 245, 3059 (1970). Disrupted GK100 cells were extracted with three volumes of phosphate buffered saline pH 7.2 containing 0.5M NaCl and applied to the column. The column was eluted with 3M NaSCN and the recovered material was analyzed by electrophoresis on polyacrylamide gel in the presence of sodium dodecyl sulfate. The results of the electrophoresis are shown in FIG. 3. Track 1 contained a mixture of molecular weight standards: a) phosphorylase B, 97,000 daltons; b) bovine serum albumen (BSA), 65,000 daltons; c) ovalbumin, 43,500 daltons; d) carbonic anhydrase, 30,000 daltons; e) soybean trypsin inhibitor, 20,000 daltons; and f) alpha-lactalbumin, 14,000 daltons. Track 3 contains yeast produced AT purified by immunoadsorption, molecular weight about 42,000 daltons. Track 7 is a sample of naturally occurring AT purchased from Sigma Chemical Company, heavily contaminated by blood proteins. A major component of Track 7 is human alpha-1-antitrypsin, molecular weight 54,000 daltons.

### EXAMPLE 6

### Activity Of Yeast Produced Alpha-1-Antitrypsin Against Serine Protease Trypsin

As a control, 10 microliters (1 microgram) of a solution of 100 microgram/ml trypsin, 100 microgram (100 microliters) of bovine serum albumin and 100 microliters of 0.05 molar TRIS, pH 8.0 buffer containing 1mM benzoylarginioyl-p-nitroanilide were mixed, and the increase in absorbance at 405 nm was measured over time in a spectrophotometer. The absorbance value of this solution was used as a standard for 100% trypsin activity. Three additional samples were run in duplicate, each containing 1 µl trypsin and, respectively containing 25 µl AT solution plus 175 µl buffer, 100 µl alpha-1-antitrypsin plus 100 µl buffer, and 200 µl alpha-1-antitrypsin. All samples contain equal concentrations of substrate and bovine serum albumin. The results demonstrated that utilizing 25 microliters of AT, 73% of the trypsin activity remained, with 100 microliters of AT, 41% of trypsin activity remained and with 200 microliters of alpha-1-antitrypsin, 26% of the trypsin activity remained. This demonstrated that by increasing the levels of the yeast made AT the trypsin inhibitory activity also increased.

### EXAMPLE 7

### Production of Alpha-1-Antitrypsin From Yeast Plasmids With Increased Genetic Stability

Increased levels of AT may be obtained by utilizing C1/l, a plasmid which is more genetically stable than CV13. The C1/l plasmid contains the entire 2-micron DNA from S. cerevisiae and, therefore, can promote its own replication and maintenance in yeast in the absence of selection for a genetic marker. Also, C1/l plasmid has a single BamHI site located in the Tc^{R} gene. Transformants carrying C1/l may be selected in E. coli by ampicillin- or tetracycline-resistance and in yeast by leucine prototrophy. C1/l contains pBR322 inserted into an EcoRI site of 2-micron DNA and carries the LEU2 gene described by J. Beggs, Nature, 275, 104-109 (1978).

The yeast TPI promoter (from CTEA32) with the synthetic DNA adaptor (described above) was inserted as a Bgl II - BamHI fragment (about 900 base pairs) into the BamHI site of C1/l. This insertion created a single BamHI site into which the human AT gene could be spliced for expression in yeast. As in the CAT1 plasmid, when the AT gene (FIG. 1A) is inserted, the resultant plasmid would have an ATG initiation codon followed by a GAG (glutamic acid codon) to allow the production of mature human AT protein sequence in yeast.

About 700 base pairs of the 3' flanking region of the yeast TPI gene was added after the human AT sequence to assist in transcription termination. The "termination" fragments are sequences from the XbaI to EcoRI sites in the plasmid TPIC10 (T. Alber and G. Kawasaki, J. Molec. Applied Genet., 1, 419-434 (1982)).

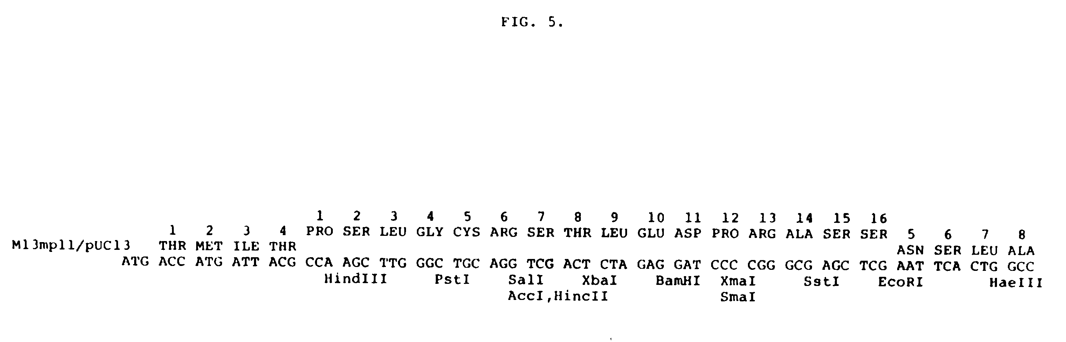

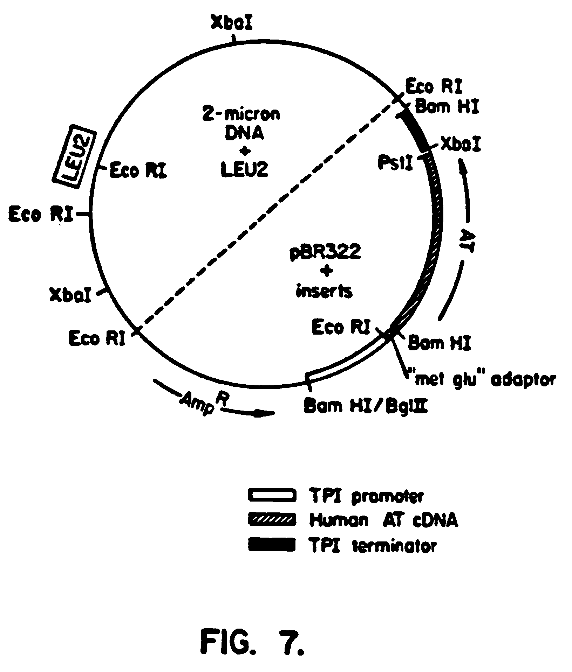

The yeast termination sequences were attached to the human AT gene by using the vector, pUC13, which has multiple cloning sites into which the terminator and AT DNA's can be separately inserted. The pUC13 plasmid was constructed as described in Vieira, J., and Messing, J., Gene, 19, 259-268 (1982) for vectors, pUC8 and pUC9. The pUC13 plasmid contained the multiple restriction site, depicted in FIG. 5, at the start of the lac Z gene. To connect the human AT gene to the TPI transcription terminator, the AT cDNA clone (FIG. 1) was inserted as a Pst I fragment into pUC13 at the single Pst I site. The AT gene was followed by an Xba I site and Eco RI site in the multiple cloning sequence. Between these Xba I and Eco RI sites of pUC13 was inserted the yeast TPI terminator as a 700 base pair Xba I-Eco RI fragment from pTPIC10. The resulting plasmid, pUCα1+FG1, contained a human AT gene with a yeast transcription terminator (See FIG. 6). An Eco RI-Bam HI synthetic DNA adaptor was then added to the Eco RI site of the plasmid, in order to create a Bam HI site on the 5' end of the yeast terminator. By using this adaptor, the human AT-yeast terminator sequence could be removed by cutting with Bam HI to liberate a fragment of approximately 2100 base pairs. This BamHI fragment was inserted into the C1/l plasmid containing the TPI promoter with BamHI adaptor. The resulting plasmid, HAT4, has the TPI promoter, ATGGAGGATCC adapter, human AT gene (from the BamHI site), and TPI terminator inserted into C1/l. The topology of HAT4 is depicted in FIG. 7.

HAT4 was transformed into N501-1B and GK100. On minimal media with 6% glucose, 2-3% of the yeast soluble protein was alpha-1-antitrypsin at a cell density of nearly 3g per liter (wet weight). Because HAT4 contained C1/l, this plasmid was maintainable in a variety of rich media, including YEPD (1% yeast extract, 2% peptone, and 2% glucose). On rich media 2-3% AT was still produced but at a higher cell density of 10-20g per liter (wet weight). The HAT4 plasmid was maintained without selection in N501-1B for over 30 divisions on rich media with greater than 70% of the cells containing the plasmid. In GK100 better than 95% of the cells had HAT4 after 30 divisions on rich media. The advantages of using HAT4 over CAT1 were 1) greater plasmid stability, 2) higher levels of AT as a percentage of total protein, 3) much greater yields of cells per liter as a result of using rich media, and 4) cheaper costs of rich media compared to synthetic (leucine-less) media. The mutant yeast strain GK100 has been placed on deposit in the American Type Culture Collection, Rockville, Maryland, ATCC No. 20669.

It is evident from the above results that yeast promoters can be efficiently used for the production of foreign proteins by regulating the expression of alien DNA in yeast. The promoters are found to be strong promoters, so as to provide for a high degree of expression. Furthermore, it would appear that the messengers are sufficiently stable as to allow for a significant degree of translation into the desired expression product. Furthermore, by employing the glycolytic promoters and appropriate nutrient media, the expression of the alien DNA can be modulated. In this way, production of the alien DNA can be turned on and off. Thus, the subject invention provides a method for using yeast as efficient host in the production of foreign proteins, where the production may be modulated. In addition, by using the glycolytic regulation gene, one can turn on and off a plurality of glycolytic promoters.

## Claims

1. A method of producing a polypeptide having the protease inhibition activity of human alpha-1-antitrypsin, comprising the steps of transforming a yeast culture with a DNA transfer vector,said vector comprising a segment coding for human alpha-1-antitrypsin and a promoter capable of controlling expression of said polypeptide in said yeast culture, and growing said culture under conditions suitable for expression of said polypeptide.

2. A method of hyperproducing a polypeptide having the protease inhibition activity of human alpha-1-antitrypsin, comprising the steps of transforming a yeast culture with a DNA transfer vector, said yeast culture comprising yeast cells which contain allelic mutations to GK-100 (ATCC 20669) mutations, said vector comprising a segment coding for human alpha-1-antitrypsin and a promoter capable of controlling expression of said polypeptide in said yeast culture and growing said culture under conditions suitable for expression of said polypeptide.

3. A method according to claim 1 or claim 2 wherein said yeast cells comprise cells of the mutant strain GX-100 (ATCC 20669).

4. A method according to any one of claims 1 to 3 wherein said vector comprises 2-micron plasmid DNA.

5. A method according to any one of the preceding claims further comprising the steps of extracting said polypeptide from said culture and purifying said polypeptide.

6. A method according to any one of the preceding claims further comprising the step of altering a suitable vector to include said segment coding for human alpha-1-antitrypsin to form said DNA transfer vector.

7. A method according to any one of the preceding claims wherein said polypeptide comprises the amino acid sequence of naturally-occurring human alpha-1-antitrypsin.

8. A method according to claim 7 wherein said polypeptide is the predominant human form of alpha-1-antitrypsin.

9. Unglycosylated human alpha-1-antitrypsin consisting essentially of an N-terminal methionine residue followed by the amino acid sequence shown in Fig 1A or 1B from amino acid 1 (Glu) to amino acid 394 (Lys).

10. Unglycosylated mature human alpha-1-antitrypsin, having the biological activity of the glycosylated form of human alpha-1-antitrypsin.

11. An unglycosylated human alpha-1-antitrypsin as claimed in claim 9 or claim 10 for introduction into a host having a deficiency of alpha-1-antitrypsin or for modulating proteolytic activity in a mammalian host.

12. An unglycosylated human alpha-1-antitrypsin as claimed in claim 9 or claim 10 for replacing alpha-1-antitrypsin inactivated by tobacco and other smoke.

## Patentansprüche

1. Verfahren zur Herstellung eines Polypeptids mit der Proteaseinhibitoraktivität von Human-alpha-1-Antitrypsin mit den Schritten: Transformierung einer Hefekultur mit einem DNA-Transfervektor, wobei der Vektor ein Human-alpha-1-Antitrypsin-codierendes Segment und einen Promotor, der in der Lage ist, die Expression des Polypeptids in der Hefekultur zu steuern, umfaßt, sowie Wachsenlassen der Kultur unter Bedingungen, die zur Expression des Polypeptids geeignet sind.

2. Verfahren zur Überproduktion eines Polypeptids mit der Proteaseinhibitoraktivität von Human-alpha-1-Antitrypsin, mit den Schritten: Transformierung einer Hefekultur mit einem DNA-Transfervektor, wobei die Hefekultur Hefezellen umfaßt, die allelische Mutationen für GK-100 (ATCC 20669) Mutationen enthalten, wobei der Vektor ein Human-alpha-1-Antitrypsin-codierendes Segment und einen Promotor, der in der Lage ist, die Expression des Polypeptids in der Hefekultur zu steuern, umfaßt, sowie Wachsenlassen der Kultur unter Bedingungen, die zur Expression des Polypeptids geeignet sind.

3. Verfahren nach Anspruch 1 oder Anspruch 2, in dem die Hefezellen Zellen des Mutantenstamms GK-100 (ATCC 20669) umfassen.

4. Verfahren nach einem der Ansprüche 1 bis 3, in dem der Vektor 2 Mikron Plasmid-DNA umfaßt.

5. Verfahren nach deinem der vorangehenden Ansprüche mit den weiteren Schritten: Extrahieren des Polypeptids aus der Kultur und Reinigen des Polypeptids.

6. Verfahren nach einem der vorangehenden Ansprüche mit dem weiteren Schritt: Verändern eines geeigneten Vektors derart, daß er daß für Human-alpha-1-Antitrypsin codierende Segment enthält, um den DNA-Transfervektor zu bilden.

7. Verfahren nach einem der vorangehenden Ansprüche, in dem das Polypeptid die Aminosäuresequenz von natürlich vorkommendem Human-alpha-1-Antitrypsin aufweist.

8. Verfahren nach Anspruch 7, in dem das Polypeptid die vorherrschende Humanform von alpha-1-Antitrypsin ist.

9. Unglykosyliertes Human-alpha-1-Antitrypsin, im wesentlichen bestehend aus einem N-terminalen Methioninrest, gefolgt von der Aminosäuresequenz, die in Figur 1A oder 1B von Aminosäure 1 (Glu) bis zur Aminosäure 394 (Lys) gezeigt ist.

10. Unglykosyliertes, vollentwickeltes Human-alpha-1-Antitrypsin mit der biologischen Aktivität der glykosylierten Form von Human-alpha-1-Antitrypsin.

11. Unglykosyliertes Human-alpha-1-Antitrypsin nach Anspruch 9 oder Anspruch 10 zur Einführung in einen Wirt mit alpha-1-Antitrypsin-Mangel oder zur Regulierung der proteolytischen Aktivität in einem Säugetierwirt.

12. Unglykosyliertes Human-alpha-1-Antitrypsin nach Anspruch 9 oder Anspruch 10 als Ersatz für durch Tabak und anderen Rauch inaktiviertes alpha-1-Antitrypsin.

## Revendications

1. Procédé de production d'un polypeptide possédant l'activité inhibitrice de protéase de l'alpha-1-antitrypsine humaine, qui comprend les étapes consistant à transformer une culture de levure avec un vecteur de transfert d'ADN, ce vecteur comprenant un segment codant l'alpha-1-antitrypsine humaine et un promoteur capable de contrôler l'expression de ce polypeptide dans la culture de levure en question, et à faire croître cette culture dans des conditions convenant pour l'expression du polypeptide.

2. Procédé d'hyperproduction d'un polypeptide possédant l'activité inhibitrice de protéase de l'alpha-1-antitrypsine humaine, qui comprend les étapes consistant à transformer une culture de levure avec un vecteur de transfert d'ADN, cette culture de levure comprenant des cellules de levure qui contiennent des mutations alléliques en mutations de GK-100 (ATCC 20669), ce vecteur comprenant un segment codant l'alpha-1-antitrypsine humaine et un promoteur capable de contrôler l'expression de ce polypeptide dans cette culture de levure, et à faire croître cette culture dans des conditions convenant pour l'expression du polypeptide.

3. Procédé suivant la revendication 1 ou la revendication 2, dans lequel les cellules de levure comprennent des cellules de la souche mutante GK-100 (ATCC 20669).

4. Procédé suivant l'une quelconque des revendications 1 à 3, dans lequel le vecteur en question comprend de l'ADN de plasmide 2 microns.

5. Procédé suivant l'une quelconque des revendications précédentes, qui comprend en outre les étapes consistant à extraire le polypeptide de la culture en question et à purifier ce polypeptide.

6. Procédé suivant l'une quelconque des revendications précédentes, qui comprend en outre l'étape consistant à modifier un vecteur convenable pour qu'il renferme le segment en question codant l'alpha-1-antitrypsine humaine afin de former le vecteur de transfert d'ADN concerné.

7. Procédé suivant l'une quelconque des revendications précédentes, dans lequel le polypeptide comprend la séquence d'aminoacides de l'alpha-1-antitrypsine humaine naturelle.

8. Procédé suivant la revendication 7, dans lequel le polypeptide est la forme humaine prédominante de l'alpha-1-antitrypsine.

9. Alpha-1-antitrypsine humaine non glycosylée, consistant essentiellement en un résidu N-terminal de méthionine suivi de la séquence d'aminoacides représentée sur la Figure 1A ou 1B depuis l'aminoacide 1 (Glu) jusqu'à l'aminoacide 394 (Lys).

10. Alpha-1-antitrypsine humaine mature non glycosylée, possédant l'activité biologique de la forme glycosylée de l'alpha-1-antitrypsine humaine.

11. Alpha-1-antitrypsine humaine non glycosylée suivant la revendication 9 ou la revendication 10, destinée à être introduite dans un hôte présentant une déficience en alpha-1-antitrypsine, ou à la modulation de l'activité protéolytique chez un hôte mammifère.

12. Alpha-1-antitrypsine humaine non glycosylée suivant la revendication 9 ou la revendication 10, destinée au remplacement d'alpha-1-antitrypsine inactivée par la fumée au tabac et d'autre fumée.
